# EUROPÄISCHE PATENTANMELDUNG

(11) **EP 1 598 073 A1**
(43) Veröffentlichungstag der Anmeldung: **23.11.2005**
(21) Anmeldenummer: 04002239.4
(22) Anmeldetag: 02.02.2004
(51) Int. Cl.: A61K 35/78, A61K 7/48

(54) **Korianderöl und dieses enthaltende Zubereitungen mit antimikrobieller und antiphlogistischer Wirkung und deren Verwendung**

(71) Anmelder: Universitätsklinikum Freiburg, 79106 Freiburg (DE)
(72) Erfinder: Augustin, Matthias, Priv.-Doz. Dr. med., 79104 Freiburg (DE); Frank, Uwe, Priv.-Doz. Dr. med., Hugstetter Strasse 55 79106 Freiburg (DE); Schempp, Christoph M.,Priv.-Doz. Dr.med.,Dipl.Biol, 79104 Freiburg (DE)
(74) Vertreter: Koepe, Gerd L.

(57) **Zusammenfassung**

Die Erfindung betrifft die Verwendung von Korianderöl zur Prophylaxe und/oder Therapie entzündlicher Erkrankungen und/oder mikrobiell bedingter oder sekundär infizierter Erkrankungen der Haut, der Schleimhäute und der Mundhöhle .

## Beschreibung

Die vorliegende Erfindung betrifft Korianderöl und dieses enthaltende Zubereitungen mit antimikrobieller und antiphlogistischer Wirkung. Die Erfindung betrifft weiter die Verwendung solcher Zubereitungen zur insbesondere topischen Prophylaxe und/oder Therapie entzündlicher, insbesondere mikrobiell bedingter oder sekundär infizierter Erkrankungen der Haut, der Schleimhäute und der Mundhöhle und zur gleichzeitigen oder gesonderten Prophylaxe und/oder Therapie von Entzündungen und Superinfektionen. Die Erfindung betrifft weiter ein kosmetisches und ein medizinisches Behandlungsverfahren.

Vor dem Hintergrund der zunehmenden Resistenzentwicklung von Mikroorganismen stellt die Behandlung mikrobiell bedingter Erkrankungen der Haut, der Schleimhäute und der Mundhöhle eine große Herausforderung in der Humanmedizin dar. Mikrobiell bedingte Hauterkrankungen werden sowohl mit antiseptischen als auch mit antibiotischen, antimykotischen oder antiviralen Medikamenten behandelt. In Abhängigkeit von der individuellen Erkrankungssituation geschieht die Applikation topisch oder systemisch. Mikrobielle Superinfektionen der Haut, wie sie beispielsweise bei der atopischen Dermatitis und dem mikrobiellen Ekzem auftreten, werden in gleicher Weise behandelt, meist in Kombination mit topischen oder systemischen Immunsuppressiva (Kortikosteroide, Immunmodulatoren). Mikrobiell bedingte Erkrankungen der Mundhöhle wie beispielsweise entzündliche gingivale und paradontale Erkrankungen, die unter anderem mit der Bildung von Plaque einhergehen, werden üblicherweise mit antiseptischen Präparaten behandelt, wie beispielsweise Chlorhexidin.

Die Nachteile dieser Behandlungsmethoden liegen zum einen in dem zum Teil hohen Nebenwirkungspotential der eingesetzten Substanzen, zum anderen in dem Umstand, dass ― im Falle von Superinfektionen - viele Mikroben inzwischen gegen Antibiotika/Antiseptika eine Resistenz entwickelt haben. Ferner verfügen Corticoide (aber auch Antibiotika/Antiseptika) nur über eine geringe Anwender- bzw. Patientenakzeptanz, was zum Teil auf den synthetischen Charakter der Substanzen, zum Teil auf die bekannten Nebenwirkungen und zum Teil auf den unangenehmen Geruch, Geschmack oder auf ein Brennen auf der Haut, auf den Schleimhäuten und in der Mundhöhle zurückzuführen ist.

Ganz anders sieht es bei zahlreichen phytotherapeutischen Substanzen aus, im Speziellen bei ätherischen (essentiellen) Ölen, die als Duft- und Geschmacksstoffe Einzug in das Alltagsleben gefunden haben und denen aufgrund ihres natürlichen Ursprungs und der bekanntermaßen kaum vorhandenen Nebenwirkungen eine hohe Akzeptanz auf Verbraucher- bzw. Patientenseite entgegengebracht wird.

Von manchen ätherischen Ölen ist bekannt, dass sie antiphlogistische, antimikrobielle und/oder antifungale Wirkungen besitzen. Ätherische Öle werden durch physikalische, bevorzugt destillative Verfahren aus Pflanzen oder Pflanzenteilen, beispielsweise auch aus Gewürzpflanzen oder deren Teilen einschließlich der Früchte, gewonnen.

Koriander (Coriandrum sativum) zählt zu der Familie der Apiaceae (Umbelliferae). Koriander ist im gesamten Mittelmeergebiet, in Mittel- und Osteuropa, Ostasien, Nord- und Südamerika verbreitet. Die bis 50 cm große, 1- bis 2-jährige Pflanze mit dünner, spindelförmiger Wurzel hat hellgrüne, gefiederte Blätter, weiß-rosa Blüten und kleine bräunlich-kugelige Samen, die außen in frischem Zustand glatt sind und mit zunehmender Trocknung meridianförmige Rippen zeigen. Korianderöl wird in der Regel durch Wasserdampfdestillation aus den getrockneten reifen Samen gewonnen.

Koriandersamen enthalten 1,5 % ätherische Öle, die vor allem aus dem Monoterpenalkohol Linalool (70 %) und ferner aus α-Pinen und anderen Terpenen (Borneol, Kampfer, Geraniol etc.) bestehen. Weitere Bestandteile der Samen sind Öle (13 bis 21 %), Phenolcarbonsäuren, Phthalide, Flavonoide und Cumarine.

Die bereits bekannte antimikrobielle und antifungale Wirkung des Korianders wird u. a. auf den Gehalt an ätherischen Ölen und Flavonoiden zurückgeführt. Neuere Studien weisen auf den schützenden Effekt von Korianderöl gegen Lipidperoxygenierung hin (Stashenko E. I. , et al., Anal. Bioanal. Chem. May 2002, 373, 1-2 : 70-74). Ferner sollen Bestandteile des Korianders durch Chelatbildung einen hemmenden Einfluß auf die Ablagerung von Blei in Knochen und Organen von Mäusen haben (Aga M., et al., J. Ethnopharmacol. Oct. 2001, 77, 2-3: 203-208).

Maruzella zeigte bereits 1959 die antibakterielle Wirkung der Korianderfrüchte gegen *E. coli, Erwinia caravora, Mycobacterium smegmatis* und *Streptomyces venezuelae,* sowie die antifungale Wirkung gegen verschiedene Pilze, wie *Epidermophyton interdigitale, Microsporum canis* u.a. (Maruzella J.C., Freundlich, M.; Nature 1959, 183: 972 -973; Maruzella J.C., Freundlich, M.; J. Am. Pharm. Assoc. 1959, 48: 356 - 358).

Für Korianderöl ist ebenfalls eine antibakterielle Wirkung gegen verschiedenste humanpathogene Infektionserreger, u.a. *Pseudomonas aeruginosa* und *E. coli,* bewiesen. Bei den Pilzen *Aspergillus aegypticus, Penicillium cyclopium* und *Trichoderma virile* wurde im Agar-Diffusionstest eine vollständige Wachstumshemmung beobachtet (Ross, S.A., El-Keltawi, N. E. Megalla, S.E.: In: Fitoterapia, 4: 201-205 (1998)). Korianderöl-Dämpfe weisen eine antibakterielle Wirkung gegen *Bacillus subtilis, Salmonella thyphosa* und besonders *Mycobacterium avium* auf.

In der Volksmedizin wird Koriander traditionell als krampflösendes Mittel, sowie gegen Blähungen und Mundgeruch eingesetzt. Ebenso soll es nervöse Spannungen und rheumatische Schmerzen lindern.

Eine Studie, bei der Mäusen über Trinkwasser und Nahrung Korianderextrakte zugeführt wurden, beweist ferner die antihyperglykämische, Insulin-freisetzende und Insulin-ähnliche Aktivität von Coriandrum sativum (Gray, A.M., Flatt P.R., (1999), Br. J. Nutr. Mar.; 81 (3) : 203-209).

Korianderöl hat eine außerordentlich hohe Verträglichkeit, und es sind kaum nennenswerte Nebenwirkungen, Kontraindikationen und Wechselwirkungen mit Arzneimitteln bekannt. So zeigen sich an chinesischen Hamsterfibroblasten bis zur cytotoxischen Konzentration von 0,125 mg/ml keine Chromosomenaberrationen (Ishidate M., Sofuni T., Yoshikawa K., Hayashi M., Nohmi T., Sawada M., Matsuoka A. (1984), Food Chem. Toxicol. 22 (8) : 623-636).

Bei Ratten, die eine fettreiche Ernährung mit Korianderöl erhalten, treten in der Leber degenerative Veränderungen (Fettzysten) und vergrößerte Nuclei der Hepatozyten auf, die durch eine unspezifische Stimulation der Hepatozyten durch die Zelldegeneration bedingt sind (Richter K.D., Mukherjee K.D., Weber N. (1996), Z. Ernährungswiss. Sept., 35 (3) : 241 - 248). Nebenwirkungen sind bei topischer Anwendung - mit Ausnahme seltener Kontaktallergien ― nicht zu erwarten und wurden im Rahmen der Studien zur vorliegenden Erfindung nicht gefunden.

In der Dermatologie, der Zahnmedizin, der Kosmetik und der Mund- und Zahnhygiene wird Korianderöl bislang lediglich als Duftstoff und Konservierungsmittel verwendet.

Aufgabe der vorliegenden Erfindung war es, natürlich vorkommende Substanzen bzw. Substanzmischungen aufzufinden, die zur Prophylaxe und Therapie entzündlicher Erkrankungen der Haut, der Schleimhäute und der Mundhöhle mit oder ohne mikrobielle Besiedlung verwendet werden können. Eine weitere Aufgabe der vorliegenden Erfindung war, natürlich vorkommende Substanzen bzw. Substanzmischungen aufzufinden, die sich zur gleichzeitigen oder gesonderten Prophylaxe und/oder Therapie von Entzündungen oder entzündlichen Erscheinungen eignen. Die unter Verwendung solcher Substanzen/Substanzmischungen erhaltenen Zubereitungen sollen keine unerwünschten Nebenwirkungen und keine Wechselwirkungen mit Arzneimitteln aufweisen, keinen unangenehmen Geruch und Geschmack haben und aufgrund der Kenntnis über das natürliche Vorkommen über eine hohe Anwender- bzw. Patientenakzeptanz verfügen.

Überraschenderweise wurde in eigenen *in-vitro-* und *in*-*vivo*-Untersuchungen nun gefunden, dass diese Aufgaben durch Korianderöl, Korianderöl enthaltende Zubereitungen und die dadurch erfolgende Verwendung von Korianderöl gelöst werden können.

Die vorliegende Erfindung betrifft Korianderöl zur gleichzeitigen oder gesonderten antiphlogistischen und antimikrobiellen Verwendung.

Die vorliegende Erfindung betrifft auch Korianderöl enthaltende Zubereitungen zur gleichzeitigen oder gesonderten antiphlogistischen und antimikrobiellen Verwendung.

Die vorliegende Erfindung betrifft auch die Verwendung von Korianderöl zur Prophylaxe und/oder Therapie mikrobiell bedingter Erkrankungen der Haut, der Schleimhäute und der Mundhöhle. Besonders bevorzugt ist dabei die durch topische Behandlung erreichbare Prophylaxe und/oder Therapie mikrobiell bedingter Erkrankungen der Haut, der Schleimhäute und der Mundhöhle.

Bevorzugte Ausführungsformen sind in den Unteransprüchen 3 bis 7 sowie 11 bis 17 beansprucht.

Die vorliegende Erfindung betrifft auch die Verwendung von Korianderöl zur Prophylaxe und/oder Therapie entzündlicher Erkrankungen. Besonders bevorzugt ist dabei die durch topische Behandlung erreichbare Prophylaxe und/oder Therapie entzündlicher Erkrankungen.

Bevorzugte Ausführungsformen sind in den Unteransprüchen 9 bis 10 sowie 11 bis 17 beansprucht.

Die vorliegende Erfindung betrifft auch die direkte Verwendung von Korianderöl zur Herstellung eines Arzneimittels und zur Herstellung einer kosmetischen Zubereitung zur insbesondere topischen Anwendung in Prophylaxe und/oder Therapie mikrobiell bedingter Erkrankungen der Haut, der Schleimhäute und der Mundhöhle.

Die vorliegende Erfindung betrifft auch die direkte Verwendung von Korianderöl zur Herstellung eines Arzneimittels und zur Herstellung einer kosmetischen Zubereitung zur insbesondere topischen Anwendung in Prophylaxe und/oder Therapie entzündlicher Erkrankungen.

Weiter betrifft die vorliegende Erfindung Verfahren zur kosmetischen Behandlung der Haut, der Schleimhäute und der Mundhöhle unter Verabreichung einer Korianderöl enthaltenden Zubereitung sowie Verfahren zur Prophylaxe und/oder Therapie mikrobiell bedingter Erkrankungen der Haut, der Schleimhäute und der Mundhöhle unter Verabreichung einer Korianderöl enthaltenden Zubereitung.

Schließlich betrifft die vorliegende Erfindung auch Verfahren zur kosmetischen Behandlung unter Verabreichung einer Korianderöl enthaltenden Zubereitung sowie Verfahren zur Prophylaxe und/oder Therapie entzündlicher Erkrankungen unter Verabreichung einer Korianderöl enthaltenden Zubereitung. Hierzu zählt auch die Behandlung irritierter und verletzter Haut sowie die Behandlung der Haut mit gestörter Barrierefunktion.

Unter Haut im Sinne der vorliegenden Beschreibung und der Ansprüche wird das den gesamten Körper bedeckende Organ verstanden.

Unter Schleimhaut im Sinne der vorliegenden Beschreibung und der Ansprüche wird die das Innere von Hohlorganen auskleidende Schicht verstanden, die durch Drüsensekrete feucht gehalten wird. Insbesondere sind damit die Mund- und Nasenschleimhaut, die Bindehaut, die Schleimhaut des Magen-Darm-Traktes und die Schleimhaut des Genitalbereichs gemeint.

Unter entzündlichen Erkrankungen werden im Rahmen der vorliegenden Beschreibung und in den Patentansprüchen Erkrankungen verstanden, bei denen es zu akuten, subakuten, chronisch rezidivierenden oder chronisch persistierenden Krankheitszuständen an Haut, Schleimhaut oder in der Mundhöhle kommt. Entzündliche Erkrankungen sind klinisch durch Rötung, Schwellung, Schmerzen, Juckreiz, Exsudation, Blasenbildung, Hyperkeratose, Hypersquamation, Erosionen, Ulzera oder andere Substanzdefekte sowie Krusten, Blasenbildung und andere Effloreszenzen gekennzeichnet. Histologisch finden sich Entzündungszellen im Corium und/oder in der Epidermis.

Unter Prophylaxe werden in der vorliegenden Beschreibung und den Ansprüchen alle Arten der Vorbeugung (Prävention) verstanden, und zwar sowohl die vorbeugende Behandlung bei gesunden Anwendern bzw. Patienten, als auch diejenige bei zu mikrobiell bedingten Erkrankung der Haut, der Schleimhäute oder der Mundhöhle neigenden Anwendern bzw. Patienten oder zu entzündlichen Erkrankungen neigenden Anwendern bzw. Patienten (Personen mit sog. "Disposition"). Weiter wird unter Prophylaxe auch die vorbeugende Behandlung bei Anwendern bzw. Patienten, die bereits einmal unter einer mikrobiell bedingten Erkrankung der Haut, der Schleimhäute oder der Mundhöhle oder unter einer entzündlichen Erkrankung litten und inzwischen die Erkrankung überwunden haben, beispielsweise durch eine erfolgreiche Behandlung wie die in der vorliegenden Beschreibung und den Ansprüchen dargestellte Behandlung (sog. "Rezidivprophylaxe"). Ferner werden unter Prophylaxe im Sinne der vorliegenden Beschreibung und der Ansprüche auch die kosmetische Behandlung sowie die pflegende und reparative Behandlung der zu Irritationen neigenden Haut verstanden.

Unter mikrobiell bedingten Erkrankungen der Haut, der Schleimhäute und der Mundhöhle im Sinne der vorliegenden Beschreibung und der Patentansprüche werden alle Arten von durch Mikroben bedingten Haut-, Schleimhäute- und Mundhöhlenerkrankungen verstanden, wie beispielsweise (jedoch nicht erschöpfend) bakteriell-eitrige Infektionen der Haut (Pyodermien; Follikulitiden, Impetigo, Erysipel) und mikrobiell-infizierte Dermatosen (Ekzeme, Akne, Psoriasis, Prurigo usw.).

Unter entzündlichen Erkrankungen oder entzündlichen Zuständen im Sinne der vorliegenden Beschreibung und der Patentansprüche werden insbesondere Erkrankungen und Zustände wie beispielsweise (jedoch nicht erschöpfend) Ekzeme (atopisches Ekzem, seborrhoisches Ekzem, allergische oder toxische Kontaktekzeme), Psoriasis, und andere hyperkeratotische Entzündungen, akute und chronische Wunden, pruriginöse Hauterkrankungen sowie seltenere Entzündungen wie Lichen ruber planus, granulomatöse und parapsoriatische Hauterkrankungen sowie die große Gruppe der Autoimmunerkrankungen mit Hautmanifestation verstanden.

Unter "gleichzeitige oder gesonderte Verwendung" wird im Rahmen der vorliegenden Beschreibung und in den Patentansprüchen verstanden, daß Korianderöl und Korianderöl enthaltende Zubereitungen sowohl eine antimikrobielle als auch eine antiphlogistische Wirkung haben und man bei der Verwendung von Korianderöl oder dieses enthaltenden Zubereitungen gemäß der Erfindung, insbesondere bei der medizinischen Verwendung von Korianderöl oder dieses enthaltenden Zubereitungen und noch spezieller bei der Verwendung von Korianderöl oder dieses enthaltenden Zubereitungen zur Herstellung eines Arzneimittels, sowohl eine antimikrobielle als auch eine antiphlogistische Wirkung erzielt und entweder die erstgenannte Wirkung eintritt, wenn eine antimikrobiell bedingte Erkrankung der Haut, der Schleimhäute und/oder der Mundhöhle vorliegt, oder die zweitgenannte Wirkung eintritt, wenn im Körper eine Entzündung vorliegt, oder beide Wirkungen gleichzeitig oder zeitlich aufeinander folgend, insbesondere zeitlich eng aufeinander folgend eintreten, wenn sowohl eine antimikrobielle Erkrankung der Haut, der Schleimhäute und/oder der Mundhöhle als auch eine entzündliche Erkrankung vorliegen. Dabei werden von der Erfindung sowohl der Fall umfaßt, daß zuerst die antiphlogistische (antiinflammatorische) Wirkung und anschließend die antimikrobielle Wirkung eintritt, als auch der Fall umfaßt, daß zuerst die antimikrobielle und anschließend die antiphlogistische (antiinflammatorische) Wirkung eintritt. Selbstverständlich können Korianderöl und auch Korianderöl enthaltende Zubereitungen auch dann eingesetzt werden, wenn es dem Anwender bzw. Patienten nur auf eine der beiden Wirkungen ankommt. Insoweit kann dann die jeweils andere Indikation von Korianderöl oder einer Korianderöl enthaltenden Zubereitung als Prophylaxe im oben definierten Sinn verstanden werden.

Erfindungsgemäß ist es möglich, Korianderöl in reiner Form einzusetzen. Reines Korianderöl ist im Rahmen der vorliegenden Beschreibung oder der Patentansprüche das aus den Koriandersamen regelmäßig durch schonende Wasserdampfdestillation gewonnene Öl, dessen wesentliche Komponenten oben genannt wurden. Unter reinem Korianderöl werden erfindungsgemäß auch solche Öle verstanden, die durch Zusammengabe der Reinbestandteile von natürlichem Korianderöl im wesentlichen in den natürlichen relativen Mengen, aber auch in davon abweichenden Mengen, erhalten werden können, unabhängig davon, ob die einzelnen Reinbestandteile selbst natürlicher oder synthetischer Herkunft sind. Unter reinem Korianderöl werden auch solche Zubereitungen verstanden, die im wesentlichen, mit Vorteil zu ≥ 90 Gew.-%, besonders bevorzugt zu ≥ 95 Gew.-%, aus Korianderöl natürlicher oder synthetischer Herkunft und zum Rest aus die Wirkung nicht beeinträchtigenden weiteren Komponenten wie beispielsweise Lösungsmittel(n) oder Resten davon oder Abbauprodukten des Korianderöls oder einzelner Komponenten davon oder auch aus anderen Komponenten ohne Einfluß auf die medizinische oder kosmetische Wirkung bestehen.

Zur Prophylaxe oder Therapie kann reines Korianderöl direkt verabreicht werden, besonders bevorzugt topisch verabreicht werden. Es ist jedoch im Rahmen der Erfindung auch denkbar, reines Korianderöl in Form Korianderöl enthaltender Zubereitungen oder Zusammensetzungen zusammen mit geeigneten anderen Stoffen, Substanzen, Trägern oder Hilfsstoffen zu verabreichen oder anzuwenden, die die Wirkungen von Korianderöl, insbesondere die antimikrobiellen und/oder antiphlogistischen Wirkungen von Korianderöl, nicht nachteilig verändern. Geeignet sind beispielsweise Lösungsmittel (z. B. Wasser, wäßrige Mischungen oder Lösungen, beispielsweise physiologische Kochsalzlösung oder Mischungen aus Wasser und Ethanol, Ethanol), Lebensmittel (z. B. Tee, Säfte, Mineralwasser), übliche Grundlagen für Sprays, Infusionen, Salben, Gele, Emulsionen (sowohl O/W-Emulsionen als auch W/O-Emulsionen) oder Pflaster.

In den Fällen, in denen Korianderöl enthaltende Zubereitungen verwendet werden, hat es sich als vorteilhaft erwiesen, wenn Korianderöl zur Prophylaxe oder Therapie in einer Konzentration von 0,1 % bis 10 %, bevorzugt in einer Konzentration von 1 % bis 8 %, noch mehr bevorzugt in einer Konzentration von 1 % bis 7 %, beispielsweise in einer Konzentration von 1 bis 6 %, verwendet wird. Die angegebenen Mengen sind bezogen auf die Gesamtmasse der verabreichten Zubereitung. Die angewendete oder verabreichte Korianderöl-Zubereitung kann geeignete andere Stoffe, Substanzen, Träger oder Hilfsstoffe umfassen, die die Wirkung von Korianderöl, insbesondere die antimikrobielle und/oder antiphlogistische Wirkung von Korianderöl, nicht nachteilig verändern. Geeignet sind beispielsweise Lösungsmittel (z. B. Wasser, wäßrige Mischungen oder Lösungen, beispielsweise physiologische Kochsalzlösung oder Mischungen aus Wasser und Ethanol, Ethanol), Lebensmittel (z. B. Tee, Säfte, Mineralwasser, Sirupe), übliche Grundlagen für Sprays, Infusionen, Salben, Gele, Emulsionen (sowohl O/W-Emulsionen als auch W/O-Emulsionen) oder Pflaster. Die Anwendung kann in jeder beliebigen Weise erfolgen; besonders bevorzugt ist erfindungsgemäß jedoch eine topische Anwendung einer Korianderöl enthaltenden Zubereitung.

Eine Anwendung kann in mehreren Gaben erfolgen, die über einen bestimmten Zeitraum verteilt sind. Wesentlicher Vorteil solcher Gaben ist, daß Korianderöl nicht toxisch ist und allgemein auch gut vertragen wird. Daher sind für die Zahl der Gaben von Korianderöl enthaltenden Zubereitungen grundsätzlich keine Grenzen gesetzt. Der Fachmann kennt jedoch aufgrund seines Fachwissens erfahrungsgemäß entwikkelte Dosen, die innerhalb eines bestimmten Zeitraums verabreicht werden, um eine vorteilhafte Wirkung gemäß der Erfindung zu erzielen. Bevorzugt werden die Zubereitungen der Erfindung mehrmals täglich verabreicht oder appliziert, und eine zweibis dreimalige Verabreichung oder Applikation pro Tag ist besonders bevorzugt.

In einer besonders bevorzugten Ausführungsform werden zusätzlich eine oder mehrere weitere antimikrobiell wirksame Substanzen und/oder antiphlogistisch wirksame Substanzen verwendet. Derartige Ausführungsformen haben den Vorteil, dass zwei oder mehr antimikrobiell und/oder antiphlogistisch wirksame Substanzen ein sich ergänzendes antimikrobielles und/oder antiphlogistisches Spektrum aufweisen können und/oder eine additive oder sogar synergistische Wirkung entfalten können. Gegebenenfalls kann auch die Menge der einzelnen Substanzen zugunsten derjenigen anderer Substanzen verringert werden. Ferner ist es durch die Verwendung von zwei oder mehr antimikrobiell und/oder antiphlogistisch wirksamen Substanzen möglich, diese für die Verwendung in der Mundhöhle geschmacklich aufeinander abzustimmen, so dass beispielsweise bei einer erwünschten starken antimikrobiellen und/oder antiphlogistischen Wirkung kein zu intensiver Koriandergeschmack auftritt. Selbstverständlich ist es auch möglich, geschmackliche Abstimmungen über den Zusatz weiterer Geschmacks- und Geruchsstoffe vorzunehmen.

Bevorzugt wird Korianderöl zur Prophylaxe und/oder Therapie von mikrobiell bedingten Hauterkrankungen, insbesondere von durch Streptokokken, Staphylokokken (einschließlich Methicillin-resistenter *Staphylococcus aureus*-Stämme), Corynebakterien, Pseudomonaden, Sproß- und Fadenpilze hervorgerufenen Hauterkrankungen verwendet (Impetigo, Wundinfektionen, Erysipel, Pyodermien, Tinea, Candidose, Akne vulgaris, etc.). Ebenso ist eine Verwendung als Prophylaxe und/oder Therapie viraler Haut bzw. Schleimhaut-Erkrankungen oder daraus resultierender Komplikationen vorgesehen (z. B. superinfizierter Herpes labialis). Ebenfalls kann Korianderöl zur Behandlung mikrobieller Keime (insbesondere Bakterien) eingesetzt werden, die unangenehme Körpergerüche generieren, beispielsweise axillären Schweiß-Geruch.

Korianderöl wird auch bevorzugt zur Prophylaxe und/oder Therapie von mikrobiell bedingten Erkrankungen der Schleimhäute, besonders bevorzugt von durch Streptokokken, Staphylokokken (einschließlich Methicillin-resistenter *Staphylococcus au*reus-Stämme, Pseudomonaden, E. coli, aber auch Sproß- und Fadenpilzen bedingten Erkrankungen der Schleimhäute, verwendet.

In weiteren bevorzugten Ausführungsformen, die dadurch bedingt sind, daß sich die Wirksamkeit von Korianderöl auch auf diese Mikroorganismen erstreckt, findet Korianderöl Verwendung bei der Prophylaxe und/oder Therapie von Erkrankungen, die hervorgerufen werden durch kommensale Mikroorganismen der Mundhöhle, die als potentiell humanpathogene Erreger gelten (beispielsweise bei intubierten Intensivpatienten, hämatologisch-onkologischen Patienten, z. B. unter Zytostatika-Therapie, bei Immunsuppression usw.). Verwendung im Bereich der Mundhöhle findet Korianderöl beispielsweise auch gegen Mikroorganismen, die an der Entstehung von kariogenen Plaques und Mundgeruch (Halitosis) maßgeblich beteiligt sind. Beispiele solcher Mikroorganismen, gegen die Korianderöl im Rahmen der Erfindung Verwendung finden kann, sind bestimmte orale Streptokokken (*S. mutans, S. mitis, S. bovis, S. agalactiae, S. constellatus, S. oralis,* Gruppe G-Streptokokken, *S. sanguis, S. intermedius, S. durans, S. salivarius, S. dysgalactiae, S. milleri, S. gordoni*), *Lactococcus, Stromatococcus, Actinomyces naeslundii, Actinomyces viscosus, Actinomyces israelii, Actinobacillus actinomycetemcomitans, Fusobacterium nucleatum, Porphyromonas gingivalis, Veillonella parvula* sowie *Capnocytophaga-, Eikenella-* und *Haemophilus*-Spezies.

Besonders bevorzugt wird Korianderöl zur Prophylaxe und/oder Therapie von teilweise mikrobiell bedingten oder superinfizierten Hauterkrankungen wie atopischer Dermatitis, mikrobiellem Ekzem, chronischem Handekzem, Psoriasis, Prurigo, Akne usw. verwendet. Noch mehr bevorzugt wird Korianderöl zur Prophylaxe und/oder Therapie von mikrobiell bedingten Hauterkrankungen wie Impetigo, Erysipel, Pyodermie, Candidiasis und Tinea verwendet.

Besonders bevorzugt ist es auch, Korianderöl zur Prophylaxe und/oder Therapie von Stomatitiden, Paradontitiden, Zahnkaries-Plaque-Bildung und Halitosis zu verwenden. Auch gegenüber gewissen, vermutlich viral bedingten Erkrankungen wie beispielsweise Aphten und Herpes labialis ist der Einsatz von Korianderöl indiziert.

Der inhibitorischer Effekt von Korianderöl auf die Entstehung von Paradontitiden, Zahnkaries verursachende Plaque-Bildung und Halitosis läßt sich *in-vivo* nachweisen. Auch gegenüber gewissen, vermutlich viral bedingten Erkrankungen (wie beispielsweise Aphten, Herpes labialis) ist eine therapeutische Wirksamkeit ersichtlich.

Von Vorteil bei der erfindungsgemäßen Verwendung von Korianderöl zur Prophylaxe und/oder Therapie von Mundhöhlenerkrankungen ist es, dass Korianderöl die an der Bildung von Plaque beteiligten oralen Bakterien inhibiert. So kann die Verwendung von Korianderöl in der Mundhöhle der Bildung von Plaque und Karies vorbeugen.

Korianderöl eignet sich außerdem zum präventiven Einsatz bei der Eradikation von multiresistenten Erregern, z.B. Methicillin-resistenten *Staphylococcus aureus*-Stämmen (MRSA-Stämmen) aus dem menschlichen Nasen-Rachenraum (Oropharynx), der ein wichtiges Reservoir für die horizontale Ausbreitung dieser Erreger in Krankenhäusern darstellt. In zahlreichen klinischen Studien konnte in den letzten Jahren nachgewiesen werden, dass durch die Elimination dieser Erreger aus dem Nasenrachenraum besiedelter Patienten und/oder Pflegepersonen die epidemiologische Ausbreitung dieser Stämme in Krankenhäusern erfolgreich unterbunden werden kann. Die derzeit eingesetzte, in einer Salbenform erhältliche antimikrobielle Substanz *"Mupirocin"* verliert leider inzwischen aufgrund der raschen Resistenzentwicklung der MRSA-Stämme zunehmend an Wirkung. Aus diesem Grunde werden derzeit von klinischen Mikrobiologen, Infektiologen und Epidemiologen fieberhaft neue Substanzen gesucht, die diese Aufgabe erfüllen können. Überraschenderweise erwies sich Korianderöl in dieser Hinsicht als außerordentlich erfolgreich. Korianderöl stellt daher eine neue, hochwirksame Alternative zur herkömmlichen Mupirocin-Salbe dar.

Im Zusammenhang mit der Prävention und/oder Therapie entzündlicher Erkrankungen werden Korianderöl und/oder Korianderöl umfassende Zubereitungen in bevorzugten Ausführungsformen der Erfindung mit Erfolg eingesetzt bei der Prävention und/oder Therapie von Ekzemen (atopisches Ekzem, seborrhoisches Ekzem, allergische oder toxische Kontaktekzeme), Psoriasis, und anderen hyperkeratotischen Entzündungen, akuten und chronischen Wunden, pruriginösen Hauterkrankungen sowie selteneren Entzündungen wie Lichen ruber planus, granulomatösen und parapsoriatischen Hauterkrankungen sowie die große Gruppe der Autoimmunerkrankungen mit Hautmanifestation.

Im Zusammenhang mit der Verwendung von Korianderöl sind dem Fachmann durch die vorliegende Erfindung keine Grenzen hinsichtlich möglicher Kombinationen von Substanzen und deren Verabreichungswege an den Patienten zur Prophylaxe und/oder Therapie mikrobiell bedingter Krankheiten der Haut, der Schleimhäute und der Mundhöhle und/oder zur Prävention und/oder Therapie entzündlicher Erkrankungen gesetzt. Erfindungsgemäß bevorzugt ist die Verwendung von Korianderöl in Kombination mit üblichen Lösungsmitteln, Träger-, Hilfs-, Füll-, Geschmacks- und/oder Geruchsstoffen und gegebenenfalls weiteren Inhaltsstoffen. Geeignete Lösungsmittel wurden oben bereits genannt und sind in bevorzugten Ausführungsformen Wasser, wäßrige (gegebenenfalls auch Elektrolyten wie anorganische oder organische Salze, z. B. Kochsalz, enthaltende) Lösungen, organische Lösungsmittel wie z. B. Ethanol oder Ethylenglycol, und Mischungen organischer Lösungsmittel (besonders bevorzugt Ethanol) mit Wasser in jedem beliebigen Verhältnis, um nur einige Beispiele zu nennen. Als Trägerstoffe kommen beispielsweise Unguentum leniens (W/O-Emulsion), DAC-Basis-Salbe, Zinci pasta mollis, Eucerin cum aqua usw. in Betracht. Bevorzugt ist Unguentum leniens. Als Hilfsstoffe kommen beispielsweise Emulgatoren oder Dispergatoren in Betracht. Geeignete Füllstoffe können beispielsweise natürliche oder synthetische Polymere oder an sich in diesem Bereich bekannte Füllstoffe sein. Als Geruchsstoffe kommen beispielsweise andere ätherische (essentielle) Öle, Lavendel- oder Kamillen-Extrakt oder Menthol in Betracht. Weitere Inhaltsstoffe können neben den oben genannten weiteren entzündungshemmenden Substanzen andere natürliche oder synthetische Komponenten sein.

Wenn Korianderöl zur Prophylaxe und/oder Therapie mikrobiell bedingter Mundhöhlenerkrankungen verwendet wird, ist es grundsätzlich möglich und erfindungsgemäß bevorzugt, aufgrund des angenehmen Geschmacks die Reinsubstanz einzusetzen. Zusätzlich kann jedoch ein/ können jedoch mehrere weitere(r) Geschmacksstoff(e) mitverwendet werden, beispielsweise Pfefferminzöl, Eukalyptusöl, Krauseminzöl, Lemongrassöl, oder Zimtöl, um nur wenige zu nennen.

Erfindungsgemäß weiter bevorzugt ist es, Korianderöl in Form einer Lösung, eines Sprays, einer Tinktur, einer Öl-in-Wasser-Emulsion oder einer Wasser-in-Öl-Emulsion zu verwenden. Bei Verwendung von Korianderöl zur Prävention, topischen Prophylaxe und/oder Therapie mikrobiell bedingter Erkrankungen der Haut ist es bevorzugt, Korianderöl in Form einer Salbe, einer Lotion, einer Creme oder eines Puders zu verwenden. Eine weitere und aufgrund ihrer exakten Dosierbarkeit bevorzugte Form der Verwendung von Korianderöl sind mikrosomale und nanosomale Zubereitungen, also Zubereitungen, die Korianderöl als solches oder in bereits mit verträglichen Komponenten verdünnter Form eingekapselt in Mikrosomen oder Nanosomen enthalten. Die Verwendung in Form einer Lösung, eines Sprays, einer Tinktur, einer Öl-in-Wasser-Emulsion, einer Wasser-in-Öl-Emulsion, einer Salbe, einer Lotion, einer Creme, einer Paste oder eines Puders umfaßt die Verwendung in Zubereitungen, die weitere Inhaltsstoffe, wie dem Fachmann bekannte Träger- und Hilfsstoffe, übliche, dem Fachmann für solche Zwecke bekannte Mittel zur Haltbarmachung und Konservierungsmittel sowie übliche, dem Fachmann für solche Zwekke bekannte Wirkstoffe, Duftstoffe, Farbstoffe etc. enthalten.

Bei Verwendung von Korianderöl zur topischen Prophylaxe und/oder Therapie mikrobiell bedingter Erkrankungen der Mundhöhle und/oder zur Prophylaxe und/oder Therapie entzündlicher Erkrankungen wird Korianderöl als antimikrobielles und gleichzeitig oder gesondert antiphlogistisches/antiinflammatorisches Mittel besonders bevorzugt in Form einer Kaumasse, eines Kaugummis, eines Bonbons, einer Pastille, einer Zahnpasta oder einer Mundspül-Lösung eingesetzt. Die Verwendung in einer Kaumasse bzw. in einem Kaugummi entspricht dabei einer besonderes bevorzugten Ausführungsform der Erfindung. Dabei ist es ganz besonders bevorzugt, Korianderöl in einer Konzentration von 0,1 % bis 10 %, weiter bevorzugt von 1 % bis 8 %, noch weiter bevorzugt von 1 % bis 7 %, beispielsweise in einer Konzentration von 1 bis 6 %, jeweils bezogen auf das Gesamtgewicht der gesamten Zubereitung, d. h. im vorliegenden Fall der Kaumasse oder des Kaugummis, d. h. beispielsweise in einer Menge von 10 mg bis 60 mg pro Gramm Kaumasse, Kaugummi, Bonbon, Pastille, Zahnpasta oder Mundspülung, zu verwenden. Die Erfindung ist jedoch nicht auf diese Konzentrationsangaben beschränkt; vielmehr kann der Fachmann Konzentrationen von Korianderöl in solchen Zubereitungen anhand seines Fachwissens und anhand weiterer Parameter (von denen das Vorhandensein weiterer Komponenten, die spezielle Darreichungsform, Wechselwirkungen mit der Umgebung, Abgabe an bestimmte Personen nur einige Beispiele sind) einstellen.

Unter Kaumasse wird in der vorliegenden Beschreibung und den Ansprüchen eine sich beim Kauen meist langsam auflösende Masse verstanden. Alternativ dazu wird unter dem Begriff Kaumasse in der vorliegenden Beschreibung und den Ansprüchen auch eine Masse verstanden, deren Träger sich im wesentlichen nicht auflöst, sondern nur Korianderöl und etwaig vorhandene weitere Wirk-, Geschmacks-, und Geruchs- und gegebenenfalls sonstige Stoffe freisetzt. Im letzen Fall spricht man im allgemeinen von einem Kaugummi. Unter dem Begriff Bonbon werden im Sinne der vorliegenden Beschreibung und der Ansprüche alle Arten von Candies, Pralinen, Lutschbonbons etc. verstanden. Die Kaumassen, Kaugummis, Bonbons und Pastillen können in den unterschiedlichsten Erscheinungsformen, Größen, Farben und Texturen vorliegen.

In einer bevorzugten Ausführungsform der Erfindung enthält die Kaumasse, das Kaugummi, das Bonbon, die Pastille, die Zahnpasta oder die Mundspüllösung zusätzlich weitere Trägerstoffe, Hilfsstoffe, Füllstoffe, Weichmacher, Inhaltsstoffe, Geschmacksstoffe und/oder Geruchsstoffe. Als Weichmacher kommen beispielsweise Gemüseöl-Produkte oder Glycerin in Betracht. Als Geschmacksstoffe können alle üblichen, dem Fachmann bekannten zuckerhaltigen oder zuckerfreien Süßungsmittel, ätherische Öle etc. verwendet werden, beispielsweise Menthol, Vanillin usw., sehr oft auch Pfefferminze.

Der moderne Kaugummi besteht aus Maissirup, Zucker, Weichmachern und Geschmacksstoffen. Die Grundsubstanz ist Harz, meistens aus der Rinde von Pinienbäumen. Man verwendet auch Chicle, eine Latexart vom Sapotill-Baum. Zum Teil wird aber auch synthetisches Material verwendet. Als Weichmacher setzt man überwiegend Gemüseölprodukte oder Glycerin ein. Der beliebteste Geschmacksstoff ist Pfefferminze. Danach rangieren Erdbeere, Apfel und Menthol. Besonders gefragt ist heute vor allem zuckerfreier Kaugummi. Er hat weniger Kalorien und bringt für die Zähne keine Kariesgefahr. Als Zuckerersatzstoffe verarbeitet man Aspartam, Mannitol und Sorbitol. Aspartam ist ein hoch konzentrierter Süßstoff; der aus der Asparaginsäure und Phenylalanin - zwei natürlichen Aminosäuren gewonnen wird. Mannitol und Sorbitol werden meistens aus Mais erzeugt. Ferner können in Kaumassen und Kaugummis alle dem Fachmann bekannten Mittel zur beschleunigten oder verzögerten Freisetzung der Inhaltsstoffe und weitere zur Mund-, Zahnfleisch- und Zahnpflege vorteilhaften Inhaltsstffe, wie beispielsweise Zahnbelag entfernende Schmirgelsstoffe, Fluoridverbindungen etc. verwendet werden.

Dabei ist es von besonderem Vorteil, dass Kaumassen, Kaugummis, Bonbons und Pastillen das enthaltene Korianderöl über einen mehr oder weniger langen Zeitraum freisetzen, der über dem Fachmann bekannte Parameter eingestellt und gesteuert werden kann. Dies ermöglicht es in bevorzugten Ausführungsformen der Erfindung, eine milde und lang anhaltende antimikrobielle und/oder antiphlogistische Wirkung zu erzielen. Der damit verbundene milde und lang anhaltende Koriandergeschmack wird darüber hinaus von den Anwendern als besonders angenehm empfunden.

Die Erfindung betrifft weiter die Verwendung von Korianderöl zur Herstellung eines Arzneimittels zur Prophylaxe und/oder Therapie, vorzugsweise zur topischen Prophylaxe und/oder Therapie, mikrobiell bedingter Erkrankungen der Haut, der Schleimhäute und der Mundhöhle. Die Erfindung betrifft auch die Verwendung von Korianderöl zur Herstellung einer kosmetischen Zubereitung zur Aufbringung auf die Haut, auf die Schleimhäute oder zur Einbringung in die Mundhöhle mit dem Ziel einer kosmetischen Wirkung.

Die Erfindung betrifft weiter die Verwendung von Korianderöl zur Herstellung eines Arzneimittels zur Prophylaxe und/oder Therapie, vorzugsweise zur topischen Prophylaxe und/oder Therapie, entzündlicher Erkrankungen. Die Erfindung betrifft auch die Verwendung von Korianderöl zur Herstellung einer kosmetischen Zubereitung zur Verwendung bei entzündlichen Erkrankungen und Zuständen mit dem Ziel einer kosmetischen Wirkung.

Weiter betrifft die Erfindung auch ein Verfahren zur kosmetischen Aufbringung oder Einbringung einer Korianderöl enthaltenden Zubereitung auf die Haut, auf die Schleimhäute oder in die Mundhöhle. Dabei bringt man die Korianderöl enthaltende Zubereitung mit der Haut, mit den Schleimhäuten oder mit dem Innern der Mundhöhle mit dem Ziel einer kosmetischen Wirkung in Kontakt. Dies kann beispielsweise geschehen durch Einnehmen (z. B. Lösungen, Emulsionen, Suspensionen, Kaumassen, Kaugummi, Bonbons), Auftragen (z. B. Lösungen, Salben, Cremes, Puder), Aufsprühen (z. B. Lösungen, Puder), Aufpinseln (z. B. Lösungen), Gurgeln (z. B. Lösungen) oder andere, dem Fachmann bekannte und geeignet erscheinende Wege. Anschließend läßt man die Korianderöl enthaltende Zubereitung für eine eine kosmetische Wirkung erbringende Zeit einwirken.

Weiter betrifft die Erfindung auch ein Verfahren zur Prophylaxe und/oder Therapie mikrobiell bedingter Erkrankungen der Haut, der Schleimhäute oder der Mundhöhle. und/oder zur Prophylaxe und/oder Therapie entzündlicher Erkrankungen. Dabei verabreicht man eine Korianderöl enthaltenden Zubereitung mit dem Ziel des Kontakts von Korianderöl und/oder seinen Derivaten und/oder seinen Metaboliten mit dem Ort der Erkrankung verabreicht, vorzugsweise der mikrobiell bedingten Erkrankung und/oder der entzündlichen Erkrankung. Dies kann beispielsweise geschehen durch Einnehmen (z. B. Lösungen, Emulsionen, Suspensionen, Kaumassen, Kaugummis, Bonbons), Auftragen (z. B. Lösungen, Salben, Cremes, Puder), Aufsprühen (z. B. Lösungen, Puder), Aufpinseln (z. B. Lösungen), Gurgeln (z. B. Lösungen) oder andere, dem Fachmann bekannte und geeignet erscheinende Wege. Anschließend läßt man das Korianderöl und/oder seine Derivate und/oder seine Metabolite für eine die Prophylaxe und/oder Therapie mikrobiell bedingter Erkrankungen sicherstellende Zeit einwirken. Vorzugsweise bringt man eine Korianderöl enthaltende Zubereitung topisch auf die Haut oder die Schleimhäute auf oder bringt diese in die Mundhöhle ein.

Die Erfindung wird nachfolgend durch Beispiele näher erläutert, die jedoch nicht der Beschränkung der Erfindung, sondern nur deren beispielhafter Erläuterung dienen.

### Ausführungsbeispiele

In einem Screening von Pflanzenwirkstoffen, die aufgrund antiphlogistischer oder antimikrobieller Eigenschaften geeignet erschienen, beispielsweise für die Anwendung bei dermatologischen Indikationen geeignet schienen, wurden neben anderen Pflanzenwirkstoffen auch Korianderöl getestet. Das Screening umfaßte:
1. Systemische Literaturanalysen
2. Die Entwicklung galenischer geeigneter Zubereitungen
3. Verträglichkeitsprüfungen bei gesunden Probanden
4. In vitro-Untersuchungen auf antimikrobielle Effekte
5. In vitro-Untersuchungen auf antiphlogistische Eigenschaften
6. Erste klinische Beobachtungen in Form von Heilversuchen
7. Human-experimentelle Untersuchungen auf antiphlogistische Wirkungen

Die Zubereitungen von Korianderöl haben sich in allen Untersuchungen als wirksam bzw. geeignet für die Anwendung bei Hauterkrankungen erwiesen, wie nachfolgende Ausführungsbeispiele zeigen.

### (1) In vitro-Studien

Korianderöl erwies sich in mehreren in vitro-Studien als stark hemmend auf verschiedene Stämme von *Staph. aureus, Enterococcus faecalis, E. coli, Pseudomonas aeruginosa* und *Candida albicans* (Tabelle 1). Wirksam war Korianderöl auch gegen MRSA (Methicillin-resistente Staphylokokken). In weiteren Untersuchungen zeigte sich eine gute Wirksamkeit gegenüber Plaque-bildenden Bakterien der Mundhöhle (u.a. *Streptococus mutans*, andere orale Streptokokken).

**Tabelle 1**

| | *S. aureus* | *E. faecalis* | *E.coli* | *P. aeruginosa* | *C. albicans* |
|---|---|---|---|---|---|
| Screening (Frank, Maier, Daschner et al., internes Manuskript 2002) | +++ | +++ | + | ++ | +++++ |

Tabelle 1: Screeningergebnis von Korianderöl in Gegenüberstellung mit Werten aus der Literatur

| | |
|---|---|
| 0 mm | Hemmung negativ |
| 1-2 mm | Hemmung (+) |
| 3-5 mm | Hemmung + |
| 6-8 mm | Hemmung ++ |
| 9-10 mm | Hemmung +++ |

### (2) Heilversuche bei entzündlichen und mikrobiellen Hauterkrankungen

In Heilversuchen wurden bislang an insgesamt 35 Patienten folgende Zubereitungen von Korianderöl eingesetzt:
1. Oleum coriandri 6,0 g in Unguentum leniens ad 100,0
2. Oleum coriandri 1,0 g in Unguentum leniens ad 100,0
3. Oleum coriandri 1,0 g in Pasta zinci mollis
4. Oleum coriandri 1,0 g in DAB-Haftpaste ad 100,0
5. Oleum coriandri 1,0 g in 5 % alkoholischer Lösung ad 100,0

In diesen Heilversuchen fanden sich günstige Verläufe und gute Ansprechraten bei chronisch-entzündlichen Dermatosen (Zubereitung 1) von erwachsenen Patienten, insbesondere bei impetigenisierten Ekzemen, bei inguinaler Candidose (Zubereitung 3) sowie bei Kindern mit Ekzemen (Zubereitung 2) und mit bakteriellen Superinfektionen (Zubereitungen 2+3). Ebenso zeigten sich Besserungen bei Patienten mit Stomatitis, Soor und Aphthen (Zubereitungen 4+5).

### (3) Antiphlogistische Effekte im UV-Erythemtest

Eine 1 % ölige Zubereitung von Oleum coriandri in Ungt. leniens reduzierte bei n = 40 Probanden UV-indizierte Erytheme auf gesunder Haut im Vergleich zur Salbengrundlage und dem unbehandelten Kontrollareal. Die Effektivität lag im Bereich von 1 % Hydrocortison (Angaben in Gew.-%).

### (4) Klinische Screening-Studie

Zubereitungen von 1 % bzw. 6 % Korianderöl in Unguentum leniens zeigten im Vergleich zur Salbengrundlage bessere Wirksamkeit bei atopischen Ekzemen und nummulären Ekzemen. Eine Zubereitung von 1 % Korianderöl in Pasta zinci mollis erwies sich als wirksam gegen Tinea-Erkrankungen sowie Candida-Intertrigo. 1 % Korianderöl in Haftpaste wurde in einer unkontrollierten Anwendung erfolgreich bei der Candida-Stomatitis und bei Aphthen eingesetzt.

Wie die obigen Ausführungsbeispiele zeigen, haben sich Korianderöl enthaltende Zubereitungen sowohl in vitro als auch klinisch als wirksam im Sinne antimikrobieller Eigenschaften und antiphlogistischer Merkmale erwiesen und sind so zur topischen Prophylaxe und/oder Therapie entzündlicher, mikrobiell bedingter oder sekundär infizierter Erkrankungen der Haut, der Schleimhäute und der Mundhöhle außerordentlich gut geeignet.

## Patentansprüche

1. Korianderöl zur gleichzeitigen oder gesonderten antimikrobiellen und.antiphlogistischen Verwendung.

2. Korianderöl enthaltende Zubereitungen zur gleichzeitigen oder gesonderten antimikrobiellen und antiphlogistischen Verwendung.

3. Verwendung von Korianderöl zur Prophylaxe und/oder Therapie mikrobiell bedingter Erkrankungen der Haut, der Schleimhäute und der Mundhöhle, vorzugsweise zur topischen Prophylaxe und/oder Therapie mikrobiell bedingter Erkrankungen der Haut, der Schleimhäute und der Mundhöhle.

4. Verwendung gemäß Anspruch 3, worin die mikrobiell bedingten Erkrankungen der Haut, der Schleimhäute und der Mundhöhle durch Gram-positive und Gramnegative Bakterien, Sproßpilze und Viren bedingte Erkrankungen sind.

5. Verwendung gemäß Anspruch 3 oder Anspruch 4 zur Prophylaxe und/oder Therapie von bakteriellen oder mykotischen Infektionen der Haut (vorzugsweise Pyodermien, Follikulitiden, Impetigo, Erysipel, Hefemykosen, Tinea) und/oder mikrobiell infizierten Dermatosen (vorzugsweise atopischer Dermatitis, mikrobiellem Ekzem, chronischem Handekzem, Psoriasis, Prurigo, Akne) und/oder mikrobiell bedingten Schleimhaut- und Mundhöhlenerkrankungen (vorzugsweise Stomatiden, Paradontitiden, Zahnkaries-Plaque-Bildung, Halitosis) und/oder viral bedingten Schleimhauterkrankungen (vorzugsweise Aphten, Herpes labialis).

6. Verwendung gemäß einem der Ansprüche 3 bis 5 zur Prävention, Prophylaxe und/oder Therapie von MRSA-Trägern und Trägern multiresistenter Erreger (Dekolonisation).

7. Verwendung gemäß einem der Ansprüche 3 bis 6, worin, wenn Korianderöl zur Prophylaxe und/oder Therapie mikrobiell bedingter Mundhöhlenerkrankungen verwendet wird, zusätzlich ein oder mehrere Geschmacksstoffe verwendet werden.

8. Verwendung von Korianderöl zur Prophylaxe und/oder Therapie entzündlicher Erkrankungen, vorzugsweise zur topischen Prophylaxe und/oder Therapie entzündlicher Erkranungen.

9. Verwendung gemäß Anspruch 8, worin die entzündlichen Erkrankungen akute, subakute, chronisch rezidivierende oder chronisch persistierende Krankheitszuständen an Haut, Schleimhaut oder in der Mundhöhle sind.

10. Verwendung gemäß Anspruch 8 oder Anspruch 9 zur Prophylaxe und/oder Therapie von Ekzemen (atopisches Ekzem, seborrhoisches Ekzem, allergische oder toxische Kontaktekzeme), Psoriasis, hyperkeratotischen Entzündungen, akuten und chronischen Wunden, pruriginösen Hauterkrankungen, Lichen ruber planus, granulomatösen und parapsoriatischen Hauterkrankungen sowie Autoimmunerkrankungen mit Hautmanifestation

11. Verwendung gemäß einem der Ansprüche 3 bis 10, worin Korianderöl in Form einer Lösung, eines Sprays, einer Tinktur, einer Öl-in-Wasser-Emulsion oder einer Wasser-in-Öl-Emulsion verwendet wird.

12. Verwendung gemäß einem der Ansprüche 3 bis 10, worin Korianderöl zur topischen Prophylaxe und/oder Therapie mikrobiell bedingter oder entzündlicher Erkrankungen der Haut in Form einer Salbe, einer Lotion, einer Creme, einer Paste oder eines Puders verwendet wird.

13. Verwendung gemäß einem der Ansprüche 3 bis 10, worin Korianderöl zur topischen Prophylaxe und/oder Therapie mikrobiell bedingter Erkrankungen oder entzündlicher Erkrankungen, vorzugsweise mikrobiell bedingter Erkrankungen oder entzündlicher Erkrankungen der Mundhöhle, in Form einer Kaumasse, eines Kaugummis, eines Bonbons, einer Pastille, einer Zahnpasta oder einer Mundspüllösung verwendet wird.

14. Verwendung gemäß Anspruch 13, worin Korianderöl in Reinform oder in einer Konzentration von 0,1 % bis 10 %, vorzugsweise in einer Konzentration von 1 % bis 8 %, weiter bevorzugt in einer Konzentration von 1 % bis 7 %, noch weiter bevorzugt in einer Konzentration von 1 % bis 6 %, bezogen auf das Gesamtgewicht an Kaumasse, Kaugummi, Bonbon, Pastille, Zahnpasta oder Mundspülung, enthalten ist.

15. Verwendung gemäß einem der Ansprüche 3 bis 14, worin Korianderöl zur Prophylaxe und/oder Therapie in Reinsubstanz oder in einer Konzentration von 0,1 % bis 10 %, bevorzugt von 1 % bis 8 %, weiter bevorzugt von 1 % bis 7 %, beispielsweise in einer Konzentration von 1 % bis 6 %, bezogen auf das Gesamtgewicht der Verabreichung, verwendet wird.

16. Verwendung gemäß einem der Ansprüche 3 bis 15, worin zusätzlich eine oder mehrere weitere entzündungshemmende und/oder antimikrobielle Substanz(en) verwendet wird/werden.

17. Verwendung gemäß einem der Ansprüche 3 bis 16, worin Korianderöl in Kombination mit üblichen Lösungsmitteln, Trägerstoffen, Hilfsstoffen, Füllstoffen, Geschmacksstoffen und/oder Geruchsstoffen und gegebenenfalls weiteren Inhaltsstoffen verwendet wird.

18. Verwendung von Korianderöl zur Herstellung eines Arzneimittels zur Prophylaxe und/oder Therapie, vorzugsweise zur topischen Prophylaxe und/oder Therapie, mikrobiell bedingter Erkrankungen der Haut, der Schleimhäute und der Mundhöhle.

19. Verwendung von Korianderöl zur Herstellung einer kosmetischen Zubereitung zur Aufbringung auf die Haut, auf die Schleimhäute und Einbringung in die Mundhöhle.

20. Verwendung von Korianderöl zur Herstellung eines Arzneimittels zur Prophylaxe und/oder Therapie, vorzugsweise zur topischen Prophylaxe und/oder Therapie, entzündlicher Erkrankungen.

21. Verfahren zur kosmetischen Aufbringung oder Einbringung von Korianderöl und/oder einer Korianderöl enthaltenden Zubereitung auf die Haut, auf die Schleimhäute oder in die Mundhöhle, bei dem man das Korianderöl und/oder die Korianderöl enthaltende Zubereitung mit der Haut, den Schleimhäuten oder dem Innern der Mundhöhle in Kontakt bringt und für eine eine kosmetische Wirkung erbringende Zeit einwirken läßt.

22. Verfahren zur Prophylaxe und/oder Therapie mikrobiell bedingter Erkrankungen der Haut, der Schleimhäute oder der Mundhöhle, bei dem man Korianderöl und/oder eine Korianderöl enthaltenden Zubereitung mit dem Ziel des Kontakts von Korianderöl und/oder seinen Derivaten und/oder seinen Metaboliten mit dem Ort der mikrobiell bedingten Erkrankung verabreicht und diese(s) für eine die Prophylaxe und/oder Therapie sicherstellende Zeit einwirken läßt, vorzugsweise worin man Korianderöl und/oder eine Korianderöl enthaltende Zubereitung topisch auf die Haut oder die Schleimhäute aufbringt oder in die Mundhöhle einbringt und diese(s) für eine die Prophylaxe und/oder Therapie sicherstellende Zeit einwirken läßt.

23. Verfahren zur Prophylaxe und/oder Therapie entzündlicher Erkrankungen, bei dem man Korianderöl und/oder eine Korianderöl enthaltenden Zubereitung mit dem Ziel des Kontakts von Korianderöl und/oder seinen Derivaten und/oder seinen Metaboliten mit dem Ort der entzündlichen Erkrankung verabreicht und diese(s) für eine die Prophylaxe und/oder Therapie sicherstellende Zeit einwirken läßt, vorzugsweise worin man Korianderöl und/oder eine Korianderöl enthaltende Zubereitung topisch aufbringt oder einbringt und diese(s) für eine die Prophylaxe und/oder Therapie sicherstellende Zeit einwirken läßt.
